# EUROPEAN PATENT APPLICATION

(11) **EP 1 977 744 A1**
(43) Date of publication of application: **08.10.2008**
(21) Application number: 07105575.0
(22) Date of filing: 03.04.2007
(51) Int. Cl.: A61K 31/197, C07C 229/08, A61P 25/08, A61P 25/18

(54) **Polymorphic Form alpha of (S)-Pregabalin and process for its preparation**

(71) Applicant: Chemo Ibérica, S.A., 08028 Barcelona (ES)
(72) Inventor: Marras, Giovanni, 28100, Novara (Novara) (IT)
(74) Representative: Barlocci, Anna

(57) **Abstract**

The present invention relates to a novel polymorphic crystalline Form α of (S)-Pregabalin, to a process for preparing it, to pharmaceutical compositions containing it, as well as its use. Crystalline Form α of (S)-Pregabalin is characterized by an X-ray powder diffraction pattern comprising peaks at about 9.5, 16.5, 17.8, 19.0, 20.0, 23.3° +/- 0.2° 2θ and a differential scanning calorimetry endothermic maximum peak at about 195°C.

## Description

### Field of the invention

The present invention relates to a novel polymorphic crystal line Form α of (S)-Pregabalin, to a process for preparing it, to pharmaceutical compositions containing it, as well as its use.

### Background of the invention

γ-Aminobutyric acid (GABA) is one of the most widely distributed inhibitory neurotransmitters involved in the regulation of brain neuronal activity. The concentration of GABA is regulated by two pyridoxal 5'-phosphate dependent enzymes: L-glutamic acid decarboxylase (GAD), which catalyzes conversion of L-glutamic acid to GABA, and GABA aminotransferase, which degrades GABA to succinic semialdehyde. When the concentration of GABA diminishes below a threshold level in the brain, convulsions may result and, conversely, raising the GABA level appears to terminate seizures.

(S)-Pregabalin increases the concentration of GABA by activating GAD and therefore is useful as a therapeutic agent for the treatment of pain, epilepsy, convulsions, psychiatric disorders, attention deficit hypersensitivity disorder, anxiety and mood disorders.
(S)-Pregabalin is a compound of Formula (I)

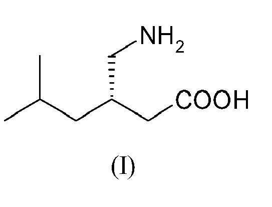

[(S)-(+)-3-aminomethyl-5-methyl-1-hexanoic acid], which is disclosed in EP 641 330 B1 with a published melting point of 175-176°C. (S)-Pregabalin is marketed under the trade name LYRICA®.
The present invention includes a novel crystalline Form α of (S)-Pregabalin.
Polymorphism is the property of some molecules and molecular complexes to assume more than one crystalline or amorphous form in the solid state. Substances are known which only appear in a single crystal form; in addition, however, there are also substances which can form two, three or even more polymorphic crystal modifications. In general, polymorphism is caused by the ability of the molecule of a substance to change its conformation or to form different inter-molecular and intramolecular interactions, particularly hydrogen bonds, which is reflected in different atom arrangements in the crystal lattices of different polymorphs. Accordingly, polymorphs are distinct solids sharing the same molecular formula, having distinct advantageous and/or disadvantageous physical properties compared to other forms in the polymorph family.
The morphology and polymorphology of organo-chemical active substances is of great importance to the chemical and pharmaceutical development thereof.
The relevant polymorphism of an organo-chemical substance is always unpredictable in respect of the number of crystal modifications, the stability thereof and their behaviour in a living organism.
The different polymorphs of a substance possess different energies of the crystal lattice and, thus, they show different physical properties of the solid state such as form, density, melting point, colour, stability, dissolution rate, milling facility, granulation, compacting etc. These differences in morphology and polymorphism may have drastic effects on the flowability of the milled solid (flowability affects the ease with which the material is handled during processing into a pharmaceutical product), development, transport stability and storage stability of individual administration forms, on the ability to produce different administration forms, on their application, on the solubility in polar or non-polar, protic or aprotic solvents, on solubility in aqueous solution, on solubility in the gastric juices, on solubility in blood serum, and finally on bio-availability. The rate of dissolution of an active ingredient in a patient's stomach fluid can have therapeutic consequences since it imposes an upper limit on the rate at which an orally-administered active ingredient can reach the patient's bloodstream. The rate of dissolution is also a consideration in formulating syrups, elixirs and other liquid medicaments. Other important properties of polymorphic forms relate to the ease of processing the form into pharmaceutical dosages, as the tendency of a powdered or granulated form to flow and the surface properties that determine whether crystals of the form will adhere to each other when compacted into a tablet. The polymorphic form may give rise to thermal behavior different from that of the amorphous material or another polymorphic form. Thermal behavior is measured in the laboratory by such techniques as capillary melting point, Differential Scanning Calorimetry (DSC) and can be used to distinguish some polymorphic forms from others. A particular polymorphic form may also give rise to distinct spectroscopic properties that may be detectable by X-Ray Powder Diffraction (XRPD).
The same also applies in respect of the physical and chemical properties of (S)-Pregabalin.

According to Chinese patent applications CN 1634869 and CN 1827590, only one crystalline Form I of (S)-Pregabalin was disclosed.
U.S. Patent Application US 20060270871 discloses a polymorphic crystalline Form I of anhydrous (S)-Pregabalin.
The crystalline form disclosed in WO 06/108151 corresponds to crystalline racemic Pregabalin hemihydrate.

The discovery of novel polymorphic forms of a pharmaceutically useful compound provides a new opportunity to improve the performance characteristics of a pharmaceutical product. It enlarges the repertoire of materials that a formulation scientist has available for designing a pharmaceutical dosage form, or a drug with a targeted release profile, or other desired characteristics, such as flowability and suitable rate of dissolution in aqueous fluid. Therefore, the preparation of novel crystalline Forms of (S)-Pregabalin is desirable.
An object of the present invention is to provide a novel polymorphic crystalline Form α of (S)-Pregabalin, a process for preparing it, pharmaceutical compositions containing it, as well as its use.

### Summary of the invention

In one embodiment, the invention provides a novel polymorphic crystalline Form α of (S)-Pregabalin. The crystalline Form α of (S)-Pregabalin is characterized by:
an XRPD pattern comprising peaks at about 9.5, 16.5, 17.8, 19.0, 20.0, 23.3° ± 0.2° 2θ; and a DSC endothermic maximum peak at about 195°C.

In another embodiment, the invention provides a process for preparing the crystalline Form α of (S)-Pregabalin by crystallising (S)-Pregabalin from at least a suitable polar solvent. The process of crystallisation comprises the steps of:
a) suspending crude (S)-Pregabalin in at least a suitable polar solvent;
b) heating until dissolution; and
c) cooling to obtain a precipitate.

In another embodiment, the invention provides a pharmaceutical composition comprising the crystalline Form α of (S)-Pregabalin, optionally together with at least one pharmaceutically acceptable excipient.

In another embodiment, the invention provides a crystalline Form α of (S)-Pregabalin for use as a medicament.

In another embodiment, the invention provides the use of the crystalline Form α for the preparation of a medicament for the prophylaxis and/or treatment of condition or disorder related to diminished concentration of GABA.

In another embodiment, the invention provides the use of the crystalline Form α for the preparation of an anti-convulsant medicament, an anti-psychotic medicament, an anti-anxiety medicament or an anti-pain medicament.

### Brief Description of the Drawings

Figure 1 provides an XRPD pattern of crystalline Form α of (S)-Pregabalin.
Figure 2 provides a DSC thermogram of crystalline Form α of (S)-Pregabalin, recorded at the conditions specified in the description. It shows an onset temperature of 191.17°C, an endothermic maximum peak at 194.69°C, with an integral of 1717.63 mJ normalized 768.17 Jg⁻¹.

### Detailed description of the invention

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

The term "about" encompasses the range of experimental error that may typically occurs in a measurement.

The term "excipient" herein means any substance, not itself a therapeutic agent, used as a carrier or vehicle for delivery of a therapeutic agent to a subject or added to a pharmaceutical composition to improve its handling or storage properties or to permit or facilitate formation of a dose unit of the composition into a discrete article such as a tablet, capsule, pill, powder, granule, pellet, lozenge, pastille, elixir, syrup, solution, suspension, emulsion, drop, lotion, spray, tincture, cream, ointment, gel, unguent, suppository and transdermal devices for oral, enteral, parenteral or topical administrations.

The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient.

We have now surprisingly found that (S)-Pregabalin may be prepared in a manner that result in a novel polymorphic form that is believed to be until now unknown. The novel polymorphic form has been designated as Form α of (S)-Pregabalin. This Form α of (S)-Pregabalin is stable at room temperature and has superior properties over Pregabalin known in the art. In particular, the Form α of (S)-Pregabalin is more soluble in water and alcohols, such as ethanol, facilitating its use in the preparation of pharmaceutical dosage forms.

DSC thermal analysis and XRPD analysis were used to characterize Form α of (S)-Pregabalin.

DSC thermal analysis was performed on a Mettler Toledo Star 822 differential scanning calorimeter. Approximately 2-5 mg samples were placed in aluminium pans and heated from 30 to 250°C in a dry nitrogen atmosphere at a heating rate of 10°C/minute.

XRPD analysis was performed on a APD 2000 Ital Structures diffractometer at room temperature, using a CuKα tube (40 kV, 30 mA, λ = 1.5418 Å) as the X-ray source. Data collection was made in 2θ step scan mode, at a scan speed of 0.02°/s in the range of 3° to 40° in 2θ. Approximately 100 mg samples were accurately grinded and placed on an aluminium sampler.

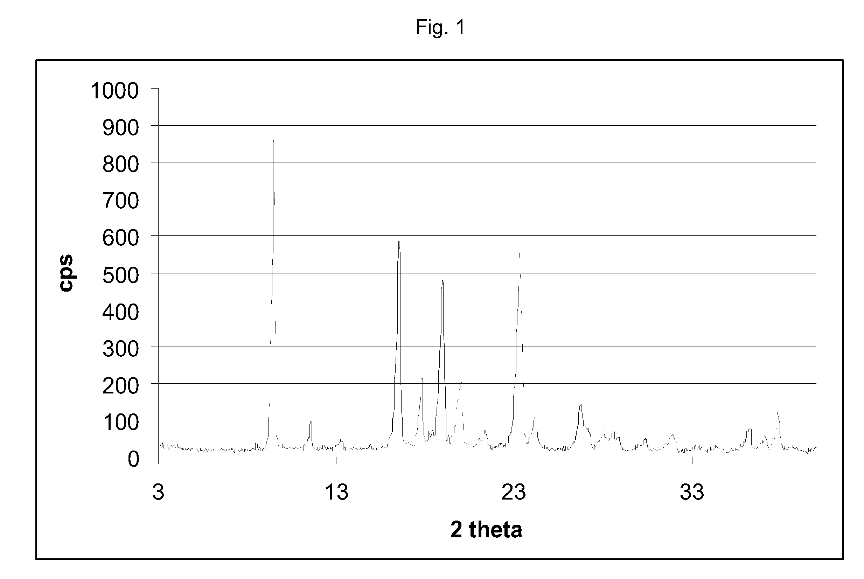

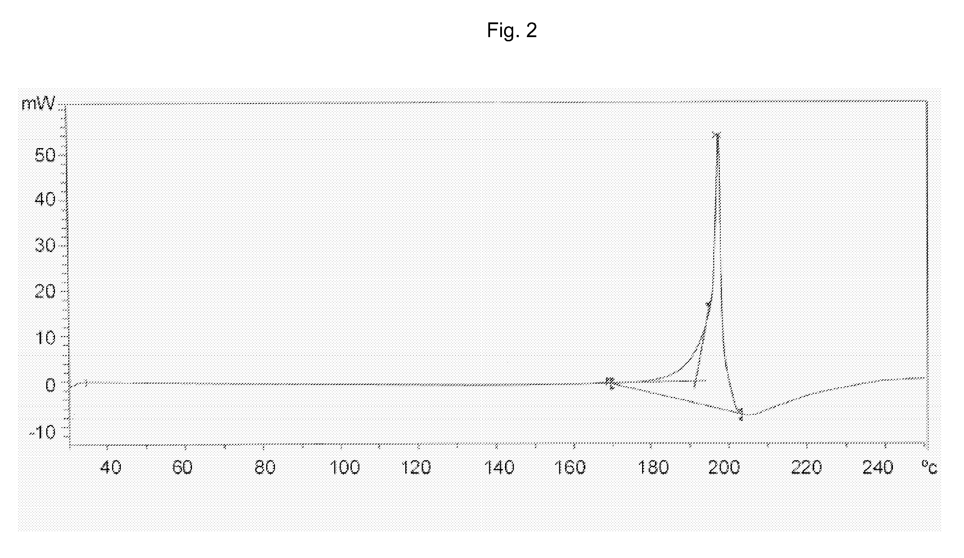

The Form α of (S)-Pregabalin has different XRPD pattern and DSC thermogram from the Pregabalin known in the art, as depicted in Figure 1 and Figure 2.

The crystalline Form α of (S)-Pregabalin has an XRPD pattern, as depicted in Figure 1, which comprises characteristic diffraction peaks at about 9.5, 16.5, 17.8, 19.0, 20.0, 23.3° ±0.2° 2θ.

Form α of (S)-Pregabalin has the characteristic DSC thermogram as depicted in Figure 2. The DSC thermogram shows a characteristic endothermic maximum peak at about 195°C.

The novel Form α of (S)-Pregabalin can also be prepared by an efficient and economic process particularly suited to large-scale preparation.

In another embodiment, the invention provides a process for the preparation of Form α of (S)-Pregabalin by crystallising (S)-Pregabalin from at least a suitable polar solvent. The process comprises the steps of:
a) suspending crude (S)-Pregabalin in at least a suitable polar solvent;
b) heating until dissolution; and
c) cooling to obtain a precipitate.

Any solvent capable of dissolving the (S)-Pregabalin and from which Form α of (S)-Pregabalin may be isolated is a suitable solvent of the invention. Suitable solvents include but are not limited to polar solvents, such as 2-pyrrolidinone, N-methyl-2-pyrrolidinone, caprolactam, and mixtures thereof. Preferred polar solvent is N-methyl-2-pyrrolidinone.

Preferably, (S)-pregabalin is in a ratio of about 1:5 to about 1:20 weight/volume to the solvents used; more preferably, (S)-pregabalin is in a ratio of about 1:10 to about 1:15 weight/volume to the solvents used.

The suspension of crude (S)-Pregabalin in a suitable polar solvent can be heated until dissolution, for example to a temperature of about 80°C to about 160°C; preferably to a temperature of about 100°C to about 140°C; more preferably to a temperature of about 120°C; still more preferably to a temperature of about 130°C. Although any convenient temperature that provides the dissolution of (S)-Pregabalin may be employed. The heating can be performed over any convenient periof of time.

The cooling can be done to a temperature of 0°C to 25°C; preferably, to a temperature of 5°C to 25°C; more preferably, the cooling is done to a temperature of about 15°C; still more preferably to a temperature of about 25°C. Although any convenient temperature that provides to obtain a precipitate of Form α of (S)-Pregabalin may be employed. The cooling can be performed over any convenient period of time, preferably over a period between 1 hour to several hours; preferably for about four hours.

One skilled in the art will appreciate that by adjusting concentration, temperature and time the yield of crystalline Form α of (S)-Pregabalin may be optimized.
Furthermore, this method may be performed at any pressure, preferably atmospheric pressure.

Crystallization of crystalline Form α of (S)-Pregabalin may also be induced by addition of a seed crystal of crystalline (S)-Pregabalin. Once crystals of Form α of (S)-Pregabalin have crystallized, either spontaneously, upon cooling, upon seeding or by another inducement, working up may be carried out generally using known procedures for the separation of the crystallized solid from the mother liquor, for example by filtration, with or without the assistance of pressure and/or vacuum, or by centrifugation, or by decantation.
The collected solid is washed with at least a solvent and dried by conventional means. The solvent may be selected between an ether, such as diethyl ether, THF or dioxane; a chlorinated hydrocarbon, such as dichloromethane or chloroform; an ester, such as ethyl acetate; an hydrocarbon, such as toluene; a ketone, such as acetone; etc. Preferred solvents are acetone, diethyl ether, ethyl acetate.

As mentioned above the compound of the invention has useful therapeutic properties.
In another embodiment, the invention provides a crystalline Form α of (S)-Pregabalin for use as a medicament.

The Form α of (S)-Pregabalin may be administered per se or, preferably, as a pharmaceutical composition.
In another embodiment, the invention provides a pharmaceutical composition comprising Form α of (S)-Pregabalin optionally together with at least one pharmaceutically acceptable excipient.

Excipients include, by way of illustration and not limitation, diluents, fillers, agglutinants, disintegrants, disintegration inhibitors, absorption accelerators, binders, carriers, suspensing/dispersing agents, film formers/coatings, adhesives, antiadherents, wetting agents, lubricants, glidants, preservatives, sorbents, surface active agents, substances added to mask or counteract a disagreeable taste or odor, flavorings, colorants, fragrances, aromatising agents, sweeteners and substances added to improve appearance of the composition.

A person skilled in the art is aware of a whole variety of such excipient compounds suitable to formulate a pharmaceutical composition. The choice of excipient will to a large extent depend on factors such as the particular mode of administration, the effect of the excipient on solubility and stability, and the nature of the dosage form.

A crystalline Form α of (S)-Pregabalin, together with a conventionally employed excipient, may be placed into the form of pharmaceutical compositions and unit dosages thereof, and in such form may be employed as solids, such as tablets, capsules, pills, powders, granules, pellets, lozenges, pastilles, or liquids, such as solutions, suspensions, emulsions, drops, lotions, sprays, tinctures, syrups, elixirs, or capsules filled with the same, all for oral use, or in the form of sterile injectable solutions for parenteral (including subcutaneous, intramuscular, and intravenous) use, or in the form of suppositories for rectal use, or in the form of creams, ointments, gels, unguents for topical use and other forms suitable for the inhalatory or transdermal administrations. Such pharmaceutical compositions and unit dosage forms thereof may comprise ingredients in conventional proportions, with or without additional active compounds or principles, and such unit dosage forms may contain any suitable effective amount of the active ingredient commensurate with the intended daily dosage range to be employed.

Pharmaceutical compositions containing a crystalline Form α of (S)-Pregabalin can be prepared in a manner well known in the pharmaceutical art and comprise at least one active compound. Generally, the compounds of this invention are administered in a pharmaceutically effective amount. The amount of the compound actually administered will typically be determined by a physician, in the light of the relevant circumstances, including the condition to be treated, the chosen route of administration, the actual compound administered, the age, weight, and response of the individual patient, the severity of the patient's symptoms, and the like.

The pharmaceutical compositions of the present invention can be administered by a variety of routes including oral, rectal, parenteral (including subcutaneous, intravenous, intramuscular), topical, transdermal, ophtalmic and intranasal. The compositions for oral administration can take the form of bulk liquid solutions or suspensions, or bulk powders. More commonly, however, the compositions are presented in unit dosage forms to facilitate accurate dosing. Typical unit dosage forms include prefilled, premeasured ampoules or syringes of the liquid compositions or pills, tablets, capsules, powders, granules, pellets, lozenges, pastilles, suppositories or the like in the case of solid compositions. In such compositions, a crystalline Form α of (S)-Pregabalin is usually a minor component (from about 0.1 to about 50% by weight or preferably from about 1 to about 40% by weight) with the remainder being various vehicles or carriers and processing aids helpful for forming the desired dosing form.

Solid forms may include, for example, any of the following ingredients, or compounds of a similar nature: a binder, such as cellulose-derived materials like powdered cellulose, microcrystalline cellulose, microfine cellulose, methyl cellulose, ethyl cellulose, hydroxyethyl cellulose, hydroxypropyl cellulose, hydroxypropylmethyl cellulose, carboxymethyl cellulose salts and other substituted and unsubstituted celluloses, gum tragacanth or gelatine; an excipient, such as starch or lactose, a disintegrating agent, such as alginic acid, primogel, or corn starch; a lubricant, such as magnesium stearate, hydrogenated castor oil, polyethylene glycol; a glidant, such as colloidal silicon dioxide; a sweetening agent, such as sorbitol, sucrose, aspartame or saccharin; or a flavoring agent, such as maltol, vanillin, menthol, citric acid, peppermint, methyl salicylate, or orange flavoring.

Liquid forms suitable for oral administration may include a suitable aqueous or nonaqueous vehicle with buffers, suspending and dispersing agents, colorants, flavors and the like.

Injectable compositions are typically based upon injectable sterile saline or phosphate-buf- fered saline or other injectable carriers known in the art. As above mentioned, a crystalline Form α of (S)-Pregabalin in such compositions is typically a minor component, with the remainder being the injectable carrier and the like.

The above described components for orally administered or injectable compositions are merely representative. Further materials as well as processing techniques and the like are set out in Part 5 of Remington's Pharmaceutical Sciences, 20^{th} Edition, 2000, Merck Publishing Company, Easton, Pennsylvania, which is incorporated herein by reference. A crystalline Form α of (S)-Pregabalin of this invention can also be administered in sustained release forms or from sustained release drug delivery systems. A description of representative sustained release materials can also be found in the incorporated materials in Remington's Pharmaceutical Sciences.

In another embodiment, the invention provides the use of crystalline Form α of (S)-Pregabalin for the preparation of a medicament for the prophylaxis and/or treatment of condition or disorder related to diminished concentration of GABA. The crystalline Form α of (S)-Pregabalin can be used, but not limited to, for the preparation of an anti-convulsant medicament, an anti-psychotic medicament, an anti-anxiety medicament, an anti-pain medicament. Compositions of the present invention are useful for, but not limited to, the treatment of pain, epilepsy, convulsions, psychiatric disorders, attention deficit hypersensitivity disorder, anxiety and mood disorders. The compound of the present invention for their therapeutic or preventive use in the above mentioned pathologies will be preferably used in a pharmaceutical composition suitable for the oral, rectal, parenteral (including subcutaneous, intravenous, intramuscular), topical, transdermal, ophtalmic and intranasal administration.

Although the most suitable administration in any given case will depend on the nature and severity of the condition being treated, the most preferred route of the present invention is oral.

While the present invention has been described in terms of its specific embodiments, certain modifications and equivalents will be apparent to those skilled in the art and are included within the scope of the present invention.

In the following, the present invention shall be illustrated by means of some examples, which are not construed to be viewed as limiting the scope of the invention.

The (S)-Pregabalin starting material used in each of the following examples can be prepared in accordance with well-known procedures. Suitable processes, without restriction, are illustratively disclosed in U.S. Pat. No. 5,637,767, WO 01/55090, WO 06/122258 or WO 06/110783.

### Example 1

### Preparation of Form α of (S)-Pregabalin

35 g of crude (S)-Pregabalin were charged at room temperature in a round bottom flask containing 400 ml of N-methyl-2-pyrrolidinone. While stirring, the mixture was heated to 120°C until a complete dissolution was observed. After cooling to 15°C over a period of four hours, the resulting mixture was filtered. The collected solid was washed with acetone, and dried under vacuum at 40°C. 26 g of Form α of (S)-Pregabalin were obtained.
Yield: 74%.

### Example 2

### Preparation of Form α of (S)-Pregabalin

20 g of crude (S)-Pregabalin were charged at room temperature in a round bottom flask containing 350 ml of 2-pyrrolidinone. While stirring, the mixture was heated to 130°C until a complete dissolution was observed. After cooling to 25°C over a period of four hours, the resulting mixture was filtered. The collected solid was washed with acetone, and dried under vacuum at 40°C. 12 g of Form α of (S)-Pregabalin were obtained.
Yield: 60%.

## Claims

1. A crystalline Form α of (S)-Pregabalin.

2. A crystalline Form according to claim 1, **characterized by** an X-ray powder diffraction pattern comprising peaks at about 9.5, 16.5, 17.8, 19.0, 20.0, 23.3° ± 0.2° 2θ.

3. A crystalline Form according to claim 2, substantially **characterized by** an X-ray powder diffraction pattern in accordance with Figure 1.

4. A crystalline Form α according to any of the preceding claims, **characterized by** a differential scanning calorimetry endothermic maximum peak at about 195°C.

5. A crystalline Form α according to claim 4, substantially **characterized by** a differential scanning calorimetry in accordance with Figure 2.

6. A process for preparing the crystalline form according to any of the preceding claims, by crystallising (S)-Pregabalin from at least a suitable polar solvent.

7. The process according to claim 6 which comprises the steps of:
a) suspending crude (S)-Pregabalin in at least a suitable polar solvent;
b) heating until dissolution; and
c) cooling to obtain a precipitate.

8. The process according to claims 6 or 7 wherein the suitable polar solvent is 2-pyrrolidinone, N-methyl-2-pyrrolidinone, caprolactam and mixtures thereof.

9. The process according to claim 8 wherein the suitable polar solvent is N-methyl-2-pyrrolidinone.

10. The process according to any of the claims 6 to 9 wherein the (S)-pregabalin is in a ratio of about 1:5 to about 1:20 weight/volume to the solvents used.

11. The process according to claim 10 wherein the (S)-pregabalin is in a ratio of about 1:10 to about 1:15 weight/volume to the solvents used.

12. The process according to claim 7 wherein the heating is performed at a temperature from about 80°C to about 160°C, preferably at a temperature from about 100°C to about 140°C.

13. The process according to claim 12 wherein the temperature is about 120°C.

14. The process according to claim 12 wherein the temperature is about 130°C.

15. The process according to claim 7 wherein the cooling is performed at a temperature from about 0°C to about 25°C, preferably at a temperature from about 5°C to about 25°C, for a period between 1 hour to several hours.

16. The process according to claim 15 wherein the temperature is about 15°C.

17. The process according to claim 15 wherein the temperature is about 25°C.

18. The process according to claim 15, wherein the cooling is performed for about 4 hours.

19. A pharmaceutical composition comprising the crystalline form according to any of the claims 1 to 5, optionally together with at least one pharmaceutically acceptable excipient.

20. A crystalline form according to any of the claims 1 to 5 for use as a medicament.

21. Use of the crystalline form according to any of the claims 1 to 5 for the preparation of a medicament for the prophylaxis and/or treatment of condition or disorder related to diminished concentration of GABA.

22. Use according to claim 21, wherein the condition or disorder is pain, epilepsy, convulsions, psychiatric disorders, attention deficit hypersensitivity disorder, anxiety and mood disorders.

23. Use of the crystalline form according to any of the claims 1 to 5 for the preparation of an anti-convulsant medicament, an anti-psychotic medicament, an anti-anxiety medicament or an anti-pain medicament.
